# EUROPEAN PATENT SPECIFICATION

(11) **EP 4 049 514 B1**
(45) Date of publication and mention of the grant of the patent: **25.10.2023**
(21) Application number: 20796786.0
(22) Date of filing: 22.10.2020
(51) Int. Cl.: H05B 6/66, H05B 6/80, H05H 9/04, H01J 25/50

(54) **MAGNETRON CONDITION MONITORING**
MAGNETRON-ZUSTANDSÜBERWACHUNG
SURVEILLANCE DE L'ÉTAT DU MAGNETRON

(30) Priority: 23.10.2019 GB 201915361
(43) Date of publication of application: 31.08.2022
(73) Proprietor: Elekta Limited, Crawley RH10 9RR (GB)
(72) Inventor: ALLEN, John, Crawley RH10 9RR (GB); SMITH, Adrian, Crawley RH10 9RR (GB)
(74) Representative: Pfundner, Benjamin Patrick
(86) International application number: PCT/EP2020/079766
(87) International publication number: WO 2021/078867

(56) References cited:
- EP-A1- 2 365 733
- WO-A2-2014/134595
- GB-A- 2 321 764
- US-A1- 2014 246 310

## Description

### FIELD

The present disclosure relates to a high-power device for supplying radiofrequency electromagnetic field to a waveguide. In particular, the disclosure relates to a magnetron coupled to a control unit in a high-power device, and a method for monitoring the magnetron in the high-power device.

### BACKGROUND

Radiotherapy is a cancer treatment that uses high doses of radiation to kill cancer cells. It utilises a radiofrequency (RF) accelerator or linear particle accelerator (linac) to provide high energy radiation to cancerous cells. This high energy radiation is produced by particles that are accelerated in the particle accelerator under strong radiofrequency fields. High-power RF is needed to produce these radiofrequency fields, and a high-power device such as a high voltage electron tube is used to produce the high peak powers. The most common choice of a high voltage electron tube is a magnetron, though other electron tubes such as Klystron are also suitable.

Magnetrons are high cost specialised components and are subject to several wear mechanisms. In general, they have a limited life span and need to be replaced during the lifetime of a particle accelerator. The cost of the magnetron may be a significant fraction of the overall cost of ownership for a machine such as a linac- of the order of 10%. The fact that it is easy to change magnetron means that any given linac may have different magnetrons installed at different points during its lifetime, and any one magnetron may have a history which is not known to the system that it is installed in.

There are certain parameters that can provide historical data for a magnetron, which, in turn, can provide predictive data pertaining to the amount of useful life remaining in the magnetron. However, existing magnetrons do not have any mechanism for recording such data, and the provenance of a magnetron installed in a linac is not always known. The fact that there is currently no mechanism that allows for traceability and condition monitoring of a magnetron once it is installed in a machine such as a linac creates uncertainties in operating the machines used in radiotherapy.

These uncertainties can give rise to the problems such as those set out below:
- Magnetrons are changed when they still have useful life left.
- It is not possible to reliably predict the end of life of a magnetron. This, in turn, may result in unplanned downtime or the necessity of stocking spare magnetrons locally.
- Magnetrons replaced in the field may have been stored in warehouses or at hospitals from an undetermined length of time. The storage creates further uncertainty because some characteristics of the magnetron degrade with storage, this can lead to confusion with long-term end of life conditions.
- It is difficult to ascertain the reasons for the failure of a magnetron with unknown provenance when it is returned under warranty or sent for repair.
- Lack of knowledge of the state of magnetrons installed in devices makes it difficult for manufacturers to predict demand for replacements over the entire installed base. This, in turn, can lead to short supply or overstocking, both of which are undesirable.

The present disclosure relates generally to a high-power device such as a magnetron system and a method for monitoring a magnetron installed in a machine such as a linac. The disclosure seeks to address the problems outlined above and those encountered in determining the amount of useful life that remains in a magnetron.

EP2365733 discloses a microwave oven which includes: heating chamber for accommodating a heating object; magnetron for heating the heating object accommodated in heating chamber; blower for cooling magnetron; temperature detection device for detecting a temperature of magnetron; and a control device for controlling an output power of magnetron on the basis of temperature information output from temperature detection device, wherein temperature detection device is disposed inside cooling fin of magnetron, and the control device controls magnetron on the basis of the temperature information obtained before cooking is started.

### SUMMARY

Aspects and features of the present invention are set out in the appended claims. The present disclosure relates to a high-power device that includes a magnetron and a control unit. Such devices are typically used in linear accelerators (linacs), which are machines that are used in radiotherapy for treating cancer patients. The high-power device of this disclosure can be used to supply radiofrequency electromagnetic field to a waveguide in a linac. While the magnetron supplies the radiofrequency field, the control unit controls the magnetron in outputting the radiofrequency energy. A magnetron typically includes a high voltage pulse connection which is enclosed in an electromagnetic compatibility enclosure and a heater or cathode connection that allows electrical connection to penetrate the enclosure. The magnetron of the present disclosure further includes a mechanism that allows the transmission of data between the magnetron and the control unit. The magnetron can thus be monitored and traced such that it is possible to reliably predict when the magnetron approaches its end of life.

Although providing magnetrons with 'tags' or chips - akin to those commonly utilised in printer ink cartridges - may be deemed reasonable solutions to the problem of monitoring and tracing magnetrons, these cannot be implemented in a simple manner due to the high-power, hostile environments in which magnetrons operate. Connecting low power digital devices such as chips directly to a high-power system can damage the low power digital device or result in the device not operating reliably. Furthermore, high voltage pulse connections to magnetrons are 'screened' in order to comply with electromagnetic compatibility (EMC) requirements. The screening encloses dangerous high voltage with earth potential for safety. As such, conventional RF tags cannot be read out when installed on magnetron so enclosed.

The mechanism that provides the means for monitoring the magnetron in this disclosure works by allowing data, typically low bandwidth data, to be reliably routed away from the high voltage environment of the magnetron to the control unit. This mechanism uses three main low-cost components: an identification chip that is mounted on the magnetron and which includes data about the magnetron, an electronic circuit that is configured to read this data, and a communication channel such as an optical fibre that routes the data away from the high voltage pulse connection of the magnetron to the control unit. In this manner, the problem of tracking a magnetron in its hostile high voltage environment is overcome by having a communication channel that routes data away from this environment.

The data that is recorded on the ID chip includes information about the provenance of the magnetron. An advantage of the present disclosure is that data on the magnetron can also be stored or cross checked by data in a cloud storage system. Given that the communication channel can operate in a bidirectional manner and can also be used to transmit cloud data or any other data to the ID chip, cloud data can be written back on the ID chip or it can be used to authenticate the data on the ID chip. The ID chip can have information such as a unique serial number, the name of a manufacturer, the manufacture date, a part number, a part revision schedule, or a manufacturer serial number. Since the data is transmitted via a communication channel, the data may be encrypted via public key encryption, and the unique serial number can form part of a private key. This ensures data security and reduces the chance of cloning the magnetron.

The electronic circuit used for reading the data on the ID chip is mounted across the terminals of the cathode or heater connection. This low power circuit can operate in the high voltage environment of the magnetron by diverting a small "ghost" power from the cathode connection to the electronic circuit. The cathode or heater connection also provides a data connection between the ID chip and the electronic circuit, thereby enabling the electronic circuit to read data from the ID chip. This setup thus circumvents the problem of having low power digital devices operating in the high voltage atmosphere of a magnetron.

The magnetron also includes a radiofrequency (RF) tuner, mounted outside the enclosure that encases the high voltage pulse connection. This RF tuner is digitally connected to the control unit. The RF tuner may be connected to the control unit via a tuner drive circuit, and this tuner drive circuit further connects the communication channel to the control unit.

The magnetron also includes an output radiofrequency transition waveguide to output the RF field to the waveguide of a linear accelerator. This transition waveguide also includes a seal that provides a dielectric gas for protecting the window of the waveguide. The magnetron also has a water connection for cooling the high-power device and a support mechanism in the form of a mechanical support.

The present disclosure thus provides a method for traceability and condition monitoring of a magnetron in a high-power device using the configuration of the high-power device as described above. Furthermore, the disclosure presents a particle accelerator which includes a high-power device according to the above description.

It would be appreciated by a person skilled in the art that since the terms "high-power" and "high voltage" are used in the context of a device that supplies a radiofrequency electromagnetic field, the terms relate to a particular voltage and power range, which is well known in the art.

### BRIEF DESCRIPTION OF THE DRAWINGS

Specific embodiments are described below by way of example only and with reference to the accompanying drawings in which:
Figure 1 illustrates an overview of a radiotherapy system
Figure 2 illustrates a high-power device that can be used in a radiotherapy machine and which includes a magnetron and a control unit
Figure 3 illustrates a high-power device where the magnetron includes a mechanism for monitoring the magnetron
Figure 4 illustrates example circuitry that is used in the mechanism for monitoring the magnetron.

### OVERVI EW

Magnetrons are essential and high cost components of particle accelerators such as linacs used in radiotherapy. They have limited lifespan and are often replaced within the lifetime of a linac. There is currently no mechanism for tracing and monitoring a magnetron, which makes it difficult for an operator to know when a magnetron is approaching its end of life. This, in turn, makes it difficult to predict machine downtime and to schedule maintenance. As a result patients may have radiotherapy sessions unduly cancelled. Due to the high voltage environment in which a magnetron operates solutions such as conventional radiofrequency identification (RFID) tags on magnetrons cannot be used to address the problem of magnetron condition monitoring and traceability. The present disclosure circumvents these problems and provides a mechanism for tracing and monitoring a magnetron, which increases the reliability of the magnetron and systems in which it is installed.

This mechanism uses three low-cost components which route data away from the magnetron to a control unit, thus making it possible for the data to be read outside the high voltage environment. These components are 1) an ID chip with memory such as an electrically erasable programmable read-only memory (EEPROM) mounted on the magnetron 2) an electronic circuit with a data connection to this ID chip so that it can read the data on the ID chip - This is mounted across the terminals of the heater connections of the magnetron but it is not connected to anything else in the high voltage environment. The power to the electronic circuit is provided through a "ghost" power from the connections to the terminals of the heater - and 3) a communication channel such as an optical fibre that is connected to the electronic circuit at one end and a tuner drive circuit at the other, which thus connects the optical fibre to the control unit.

In this way, the ID chip on the magnetron can record any information about the magnetron, the electronic circuit, which is a low power device, can read this data and is further able to operate in a high voltage environment by virtue of being only connected to the terminals of the magnetron's heater connections, and the fibre optic connection transmits the data away from the magnetron to the control unit. In this way a mechanism is provided for tracing and monitoring a magnetron.

### SPECIFIC DESCRIPTION OF CERTAIN EXAMPLE EMBODIMENTS

Radiotherapy machines are important tools in modern cancer treatment. They are large, complex machines, with many moving parts and inter-operating mechanisms. Despite precision engineering and rigorous testing, some component parts of a radiotherapy machines may start to degrade over the lifetime of the machine. This can sometimes lead to sub-optimal operation and even the occasional safety override. One of the key components that is susceptible to degradation is the magnetron.

There is currently no routine mechanism for recording data from the magnetron of a system such as a linac used in radiotherapy. Furthermore, the behaviour of a magnetron over its lifetime is not tracked or recorded. Particularly, conditions that a magnetron has been exposed to, or the behaviour of the magnetron are not tracked or analysed. The lack of the ability to correlate magnetron data with linac data, means that a richer source of information is not available. As a result, there is no known way of identifying magnetrons which are approaching the end of their operational life. This can be particularly problematic when a magnetron is replaced within a device and the magnetron's provenance is not known.

Not having a mechanism for recording information on, and monitoring of, a magnetron has undesirable consequences. Without the ability to predict the end of life for a magnetron, magnetrons may be replaced while they are still operational. This is, of course, has financial disadvantages for the owner of the radiotherapy device. Manufacturers are also at a disadvantage because they are not able to predict demand for magnetrons, which can lead to shortages or overstocking. Not being able to predict a magnetron's end of life can also entail unplanned downtime, which gives rise to uncertainties, both for owners and patients. Furthermore, spare magnetrons that have long storage life may prove to have shorter lifespans than anticipated, which gives rise to further uncertainties.

The present application seeks to overcome these problems through devices that are capable of recording information related to a magnetron and methods for monitoring the condition of the magnetron using the recorded information. The disclosed devices and methods are advantageous as they allow a manufacturer or maintenance service provider to predict the end of life of a magnetron, thereby facilitating timely maintenance, replacement, and manufacture of magnetrons. The disclosed methods help to reduce unplanned downtime and thus minimise disruption to the machine's normal operation. The disclosed devices further facilitate more accurate assessment of reasons for failure when a magnetron is sent for repair. This, in turn, provides the necessary feedback to manufacturers for implementing improvements in magnetrons.

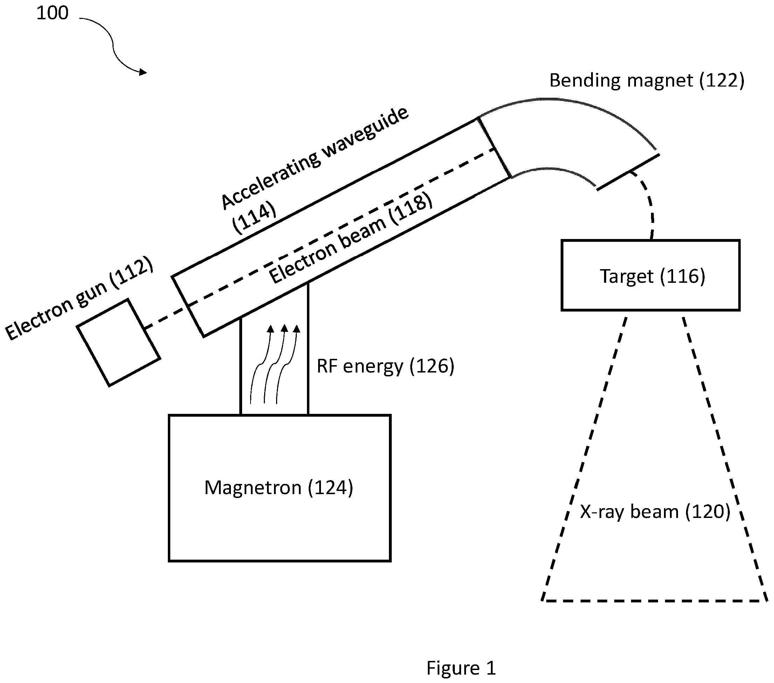

Figure 1 illustrates a high-level overview of a particle accelerator, such as those utilised in radiotherapy. The device is a linear accelerator (linac) 110 which includes a source of electrons 112, a waveguide 114, and a target 116. Electrons are emitted from the source 112 and are accelerated through the waveguide 114 along an acceleration path which is coincident with the centre axis of the waveguide. The electron beam 118 is bent using magnets 122 and strikes the target 116, to produce an x-ray beam 120. The x-ray beam 120 is used to treat patients. The linac 110 also includes a magnetron 124, which generates radiofrequency (RF) electromagnetic waves 126 to accelerate charged particles to high energies along an acceleration path. The electron beam 118 may be used to treat the patient directly, or may be directed towards a target 116, which produces high energy x-rays 120.

The source of electrons 112 may be an electron gun. The source of electrons is configured to inject electrons into the waveguide 114. The waveguide 114 comprises a plurality of interconnected acceleration cavities (not shown) forming a channel through which the electron beam passes. The injection of electrons into the waveguide 114 is synchronised with the pumping of RF waves 126 into the waveguide 114. The design and operation of the magnetron 124, electron source 112, and the waveguide 114 are such that the RF waves 126 accelerate the electrons to very high energies as they propagate through the waveguide 114 down the acceleration path 118. The waveguide is designed in order that a suitable electric field pattern is produced which accelerates electrons propagating through the waveguide 114.

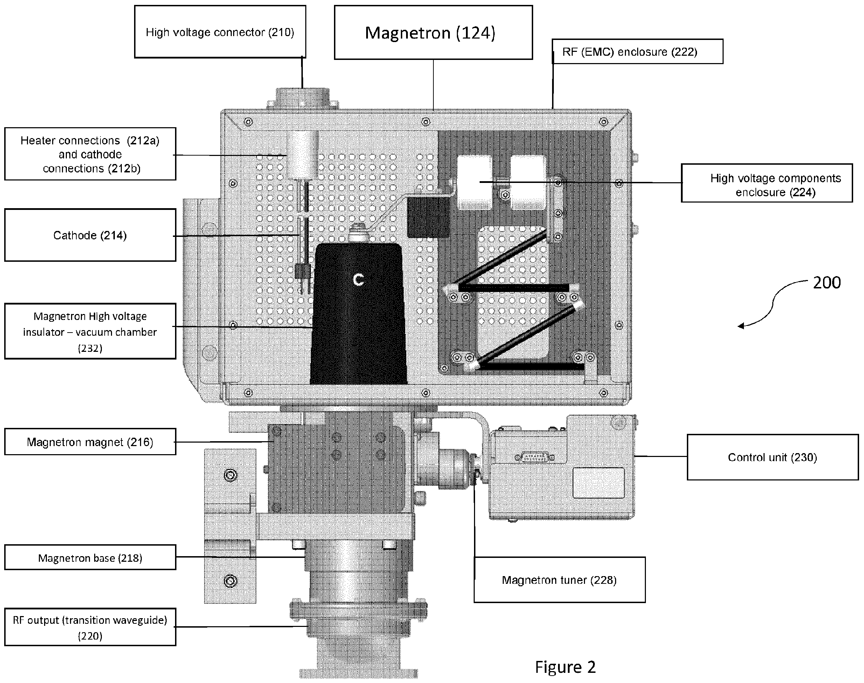

The device 200 of Figure 2 shows a schematic cross-sectional diagram of magnetron 124 and its connection to control unit 230 of a linac. The components of the magnetron 124 are drawn as disparate entities and the connections are not necessarily shown so as to simplify their representation. The way in which the different components are connected, along with their functions, are detailed in the ensuing paragraphs. The magnetron is comprised of a high voltage connector 210, which is enclosed in an electromagnetic compatibility (EMC) shield 222 in order to comply with electromagnetic compatibility (EMC) requirements. This encloses dangerous high voltage with earth potential for safety. The magnetron also includes a heater or cathode 214. One way to allow electrical connection to penetrate inside the enclosure 222 is by providing cathode connections 212b and heater connections 212a inside the high voltage connector 210, although there are other possible alternative arrangements. The cathode 214 is placed inside a vacuum chamber 232 - this is not visible in the diagram as it is enveloped in a high voltage insulator. The vacuum chamber is made of copper and ceramic components that extend from just below the high voltage insulator (typically a plastic cover) to an RF ceramic window (which cannot be seen as its inside the magnetron base). This is coupled to a magnet 216 which provides a magnetic field. This magnet can be an electromagnet or a permanent magnet. A transition waveguide 220 outputs the RF field. The magnetron further includes an RF tuner 228, which is mounted outside the enclosure 222 and is digitally connected to a control unit 230. The control unit 230 also controls the linac. The magnetron further comprises water connections for cooling, and a mechanical support (not shown).

The operation of magnetron 124 is now described with reference to Figure 2. In magnetron 124, high voltage connection 210 provides a high negative potential to the cathode 214 in very short pulses. This is in contrast to the power provided to the heater, which is supplied continuously at a much lower voltage (typically less than 20V). The cathode 214, which emits electrons, is placed inside the vacuum chamber 232. The chamber comprises cavities (not shown) around its outer rim such that when electrons spiral outward in a circular path under the influence of the magnetic field of magnet 216, they sweep past through these cavities. The cavities act as resonators, leading to a resonant RF field being induced in the cavities. This RF field is then outputted through the transition waveguide 220. Magnetron tuner 228 is used to tune the resonant frequency of the magnetron 124.

The control unit 230 of device 200 also controls the other components of the linear accelerator 100. Controlling the other components can include: controlling the electron gun 112 to feed electrons to the waveguide 114, controlling the gantry (not shown) to rotate according to a patient's treatment plan to provide the angle at which radiation is delivered to the patient, and controlling a collimator (not shown), such as a multi leaf collimator, MLC, to collimate the beam 120 according to the treatment plan.

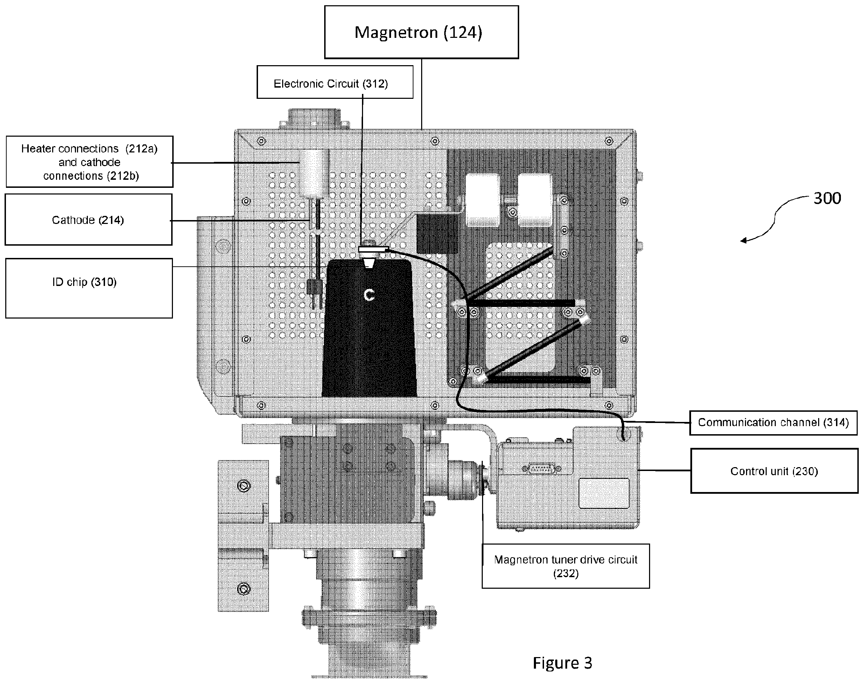

Figure 3 illustrates an advantageous embodiment of the present disclosure, which provides a mechanism for integrating the necessary interfaces in order to enable the recording of information about magnetron 124 and monitoring the magnetron. This mechanism is configured to transmit data between the magnetron 124 and the control unit 230. The mechanism includes an identification (ID) chip 310 mounted on the magnetron 124, an electronic circuit 312 mounted across the terminals of the heater connections 212a, and a communication channel 314 such as an optical fibre, connected between the magnetron 124 and a tuner drive circuit 232, which is, in turn, connected to the control unit 230. There is also a data connection (not shown) between the electronics circuit 312 and the ID chip 310.

In Figure 3, the ID chip 310 is configured to record data related to the magnetron 124. This data can, for example, be a unique serial number, and/or additional information about the magnetron such as the manufacturer, the manufacture date, part number, part revision date, manufacturer serial number, etc. The electronics circuit 312, which is a low voltage device, is mounted across the heater connections 212a, and is configured to read the data from the ID chip 310. Given that the cathode connections 212b are high voltage terminals, there are no electrical connections to the electronics circuit board 312 apart from the two heater connections and the data connection to the ID chip 310. Power to the electronics circuit board 312 is provided by diverting a small amount of the power from the heater connection 212a to the circuit board 312. A communication channel 314 such as an optical fibre is configured to route data from the electronic circuit 312 to a tuner drive circuit 232, which is connected to the control unit 230. In this way, data is routed away from the high voltage area of the magnetron 124 to the control unit 230 where it can be easily read to provide information about the magnetron 124. Alternatively, electronic circuit 312 may be incorporated into the magnetron 124, where the ID chip 310 may be part of electronic circuit 312.

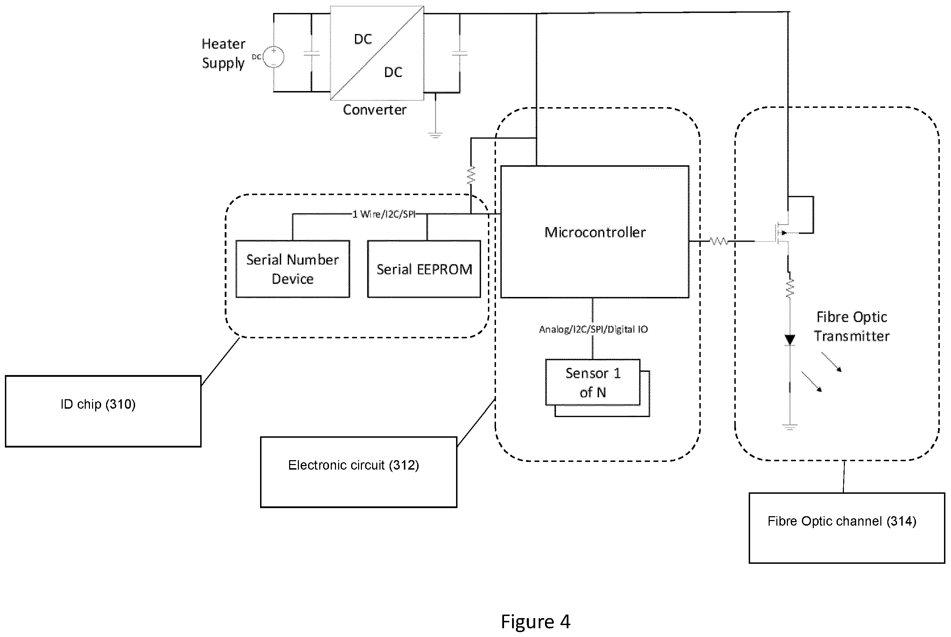

Figure 4 illustrates a schematic drawing that shows how the ID chip 310, the electronic circuit board 312, and the fibre optic channel 314 are connected. As can be seen in this diagram, the electronic circuit board 312 draws power from the heater connections 212a and includes a microcontroller and a sensor array. The circuit 312 is arranged to read data from the ID chip 310, which, in turn, includes an electrically erasable programmable read-only memory (EEPROM) on which data can be recorded and from which data can be transmitted, and information such as the device serial number. The electronic circuit board is further connected to fibre optic channel 314, which then transmits the data to the control unit 230.

The advantageous embodiment of Figure 3 thus provides means for recording information related to a magnetron and thereby enables traceability and monitoring of a magnetron. In this manner, small, low-cost components are utilised to record data about the magnetron, read this data, and transfer the data away from the high voltage environment of the magnetron to the linac's control unit. The data from the magnetron, which is read by the magnetron tuner drive circuit 232, provides vital information about the provenance of the magnetron. This information enables a manufacture to reliably predict the end of life of a magnetron, which, in turn, reduces unplanned downtime. The information also reduces the need to stock magnetrons locally, or to use magnetrons that have long storage life. The data can further provide information about previously existing faults in the magnetron, thus making it easier to determine with confidence causes of future failures. The data is also useful for manufacturers because it provides global knowledge of the state of the installed magnetrons, which, in turn, makes it possible to project future needs for magnetron replacements. This reduces the risk of shortages or overstocking of magnetrons.

Data that is transmitted through the communication channel 314 is low bandwidth data and an optical fibre can be used to transmit the data. The maximum amount of data that is transmitted is less than the maximum capacity of the memory of the ID chip and the low bandwidth data is typically in the order of a few kilo bits per second - typically less than 3000 bits per second. This means that the data can be easily fit into a 19200K Baud serial channel. The bandwidth can be even lower because, for example, the manufacturer's serial number and the unique serial number can be sent at much lower frequencies but sending all the data together makes the design easier. In some embodiments of the disclosure, the communication channel 314 can be bidirectional to allow data to be written back on the ID chip 310. This is done using the tuner drive circuit 232, which can be configured to transmit data back to the ID chip 310 through the communication channel 314. The data uniquely identifies the magnetron 124 and may be stored on the ID chip 310. The unique identifying features of the magnetron 124 may also be used as a key to the data stored elsewhere. The stored data can pertain to information such as the number of hours that the magnetron has operated under high or low tension, the linac's ID, and the date of the magnetron's installation. An important feature of the stored data is that some of the data is linked to the magnetron and some is linked to the linac. This data becomes more valuable when the two sets of data (related to magnetron and linac) are linked. For example, if the linac 100 detects that the magnetron's chip 310 has changed then the system knows that a new magnetron has been installed, and can log this information. If this is connected to a cloud service, the system is then able to verify that the magnetron 124 is new and record other information such as the credentials of the installing engineer. In this way, the magnetron data that can be regularly updated, which further facilitates condition monitoring and traceability of the magnetron.

The ID chip 310 includes an electrically erasable programmable read-only memory (EEPROM) on which data can be recorded and from which data can be transmitted. The EEPROM can be encrypted via public key encryption in order to ensure data security. In this way, the ability to clone is limited using the unique serial number as part of the private key. Additional useful data such as the local temperature can be transmitted back via the communication channel 314 and recorded on the ID chip 310.

As well as providing a mechanism for monitoring a magnetron, the present disclosure also provides a method for traceability and conditioning of a magnetron using this mechanism. In this method, data related to the magnetron is recorded on an ID chip and the ID chip is placed on the magnetron. The data is then read by an electronic circuit from where it is communicated to a control unit outside the magnetron using a communication channel such as an optical fibre. The magnetron is then monitored based on this data.

The data may be correlated with stored data on the magnetron or on a cloud base repository. The data may, for example, be a unique serial number, the name of the manufacturer, the manufacture date, a part number, a part revision schedule, or a manufacturer serial number. In order to ensure data security, the data may be encrypted via public key encryption and the unique serial number can form a part of a private key.

The high-power device described in this disclosure can be used in conjunction with an acceleration waveguide in a particle accelerator such as those utilised in radiotherapy.

It will be understood that the above description is of specific embodiments by way of aspect only and that many modifications and alterations will be within the skilled person's reach and are intended to be covered by the scope of the appendant claims.

## Claims

1. A high-power device for supplying a radiofrequency electromagnetic field to a waveguide, the device comprising:
a magnetron (124) configured to supply a radiofrequency electromagnetic field to a waveguide; and,
a control unit (230) configured to control the magnetron to output radiofrequency energy to the waveguide, wherein:
the magnetron comprises:
a high voltage pulse connection (210) enclosed in an enclosure (222);
a heater connection (212) configured to allow an electrical connection to penetrate the enclosure; and
a mechanism configured to transmit data between the magnetron and the control unit, **characterised in that** the mechanism comprises:
an identification chip (310) configured to include the data, wherein the data is about the magnetron;
an electronic circuit (312) configured to read the data on the identification chip; and,
a communication channel (314) configured to route the data away from the high voltage pulse connection to the control unit.

2. The device of claim 1, wherein the device further comprises at least one of stored data on the high-power device or stored data on a cloud base repository wherein the stored data includes a history of the magnetron.

3. The device of claim 1 or 2, wherein the communication channel is a fibre optic connection.

4. The device of any preceding claim, wherein the electronic circuit is mounted across terminals of the heater connection, and wherein the heater connection is configured to provide power to the electronic circuit.

5. The device of any preceding claim wherein the heater connection provides a connection between the electronic circuit and the identification chip

6. The device of any preceding claim , wherein the enclosure comprises an electromagnetic compatibility (EMC) shield.

7. The device of any of the preceding claims wherein at least one of:
the data is low bandwidth data;
the data comprises at least one of: a unique serial number; a name of a manufacturer; a manufacture date; a part number; a part revision schedule; a manufacturer serial number; and
the data is encrypted via public key encryption, and wherein the unique serial number is part of a private key.

8. The device of claim 3 wherein the fibre optic connection is further configured to allow data transmission from the control unit to the high voltage pulse connection.

9. The device of any of the preceding claims wherein the magnetron further comprises at least one of: a permanent magnet; an electromagnet, water connections for cooling and a mechanical support.

10. The device of any of the preceding claims wherein the magnetron further comprises a radio frequency (RF) tuner, wherein the RF tuner is mounted outside the enclosure and is digitally connected to the control unit; and a radio frequency (RF) tuner, wherein the RF tuner is mounted outside the enclosure and is digitally connected to the control unit via a tuner drive circuit, and wherein the fibre optic is connected to the control unit via the tuner drive circuit;
an output radio frequency (RF) transition waveguide to output the RF field to the waveguide, and an RF transition waveguide seal, wherein the RF transition waveguide seal provides a dielectric gas for protecting a window of the waveguide.

11. A method of providing traceability and condition monitoring of a magnetron in a high-power device, the method comprising:
recording data related to the magnetron on an identification chip, wherein the identification chip is placed on the magnetron;
reading the data by an electronic circuit;
communicating the data to a control unit in the high-power device; and
based on the data, monitoring condition of the magnetron.

12. The method of claim 11, wherein the method further comprises correlating the data with at least one of stored data on the high-power device or stored data on a cloud base repository, wherein the stored data includes a history of the magnetron.

13. The method of claim 11 or claim 12, wherein the data comprises at least one of: a unique serial number; a name of a manufacturer; a manufacture date; a part number; a part revision schedule; a manufacturer serial number, and/or wherein the data is encrypted via public key encryption, and wherein the unique serial number is part of a private key.

14. The method of any of claims 11 to 13 wherein the communicating the data is performed with an optical fibre.

15. A particle accelerator comprising:
a waveguide for accelerating charged particles along an acceleration path; and the high-power device of any of claims 1 to 10.

## Patentansprüche

1. Hochleistungsvorrichtung zum Versorgen eines Wellenleiters mit einem elektromagnetischen Hochfrequenzfeld, wobei die Vorrichtung umfasst:
ein Magnetron (124), das konfiguriert ist zum Versorgen eines Wellenleiters mit einem elektromagnetischen Hochfrequenzfeld; und
eine Steuereinheit (230), die konfiguriert ist zum Steuern des Magnetrons, um eine Hochfrequenzenergie an den Wellenleiter auszugeben, wobei:
das Magnetron umfasst:
eine Hochspannungspulsverbindung (210), die in einem Gehäuse (222) eingeschlossen ist;
eine Heizelementverbindung (212), die konfiguriert ist zum Erlauben, dass eine elektrische Verbindung in das Gehäuse eintritt; und
einen Mechanismus, der konfiguriert ist zum Übertragen von Daten zwischen dem Magnetron und der Steuereinheit,
**dadurch gekennzeichnet, dass** der Mechanismus umfasst:
einen Identifizierungschip (310), der konfiguriert ist, um die Daten zu beinhalten, wobei die Daten das Magnetron betreffen;
eine elektronische Schaltung (312), die konfiguriert ist zum Lesen der Daten in dem Identifizierungschip; und
einen Kommunikationskanal (314) der konfiguriert ist zum Weiterleiten der Daten weg von der Hochspannungspulsverbindung zu der Steuereinheit.

2. Vorrichtung nach Anspruch 1, wobei die Vorrichtung außerdem mindestens eine umfasst von in der Hochleistungsvorrichtung gespeicherten Daten oder von an einem Cloud-Basisaufbewahrungsort gespeicherten Daten, wobei die gespeicherten Daten eine Historie des Magnetrons beinhalten.

3. Vorrichtung nach Anspruch 1 oder 2, wobei der Kommunikationskanal eine Glasfaserverbindung ist.

4. Vorrichtung nach einem der vorhergehenden Ansprüche, wobei die elektronische Schaltung über die Klemmen der Heizelementverbindung montiert ist und wobei die Heizelementverbindung konfiguriert ist zum Bereitstellen einer Energie für die elektronische Schaltung.

5. Vorrichtung nach einem der vorhergehenden Ansprüche, wobei die Heizelementverbindung eine Verbindung zwischen der elektronischen Schaltung und dem Identifizierungschip bereitstellt.

6. Vorrichtung nach einem der vorhergehenden Ansprüche, wobei das Gehäuse eine elektromagnetische Verträglichkeitsabschirmung (Electromagnetic Compatibility shield, EMC-Abschirmung) umfasst.

7. Vorrichtung nach einem der vorhergehenden Ansprüche, wobei mindestens eines gilt von:
die Daten sind Daten mit niedriger Bandbreite;
die Daten umfassen mindestens einen von: einer eindeutigen Seriennummer; einem Namen eines Herstellers;
einem Herstellungsdatum; einer Artikelnummer; einem Bauteilprüfplan; einer Seriennummer des Herstellers; und die Daten werden über eine Verschlüsselung mit öffentlichem Schlüssel verschlüsselt, und wobei die eindeutige Seriennummer Teil eines privaten Schlüssels ist.

8. Vorrichtung nach Anspruch 3, wobei die Glasfaserverbindung außerdem konfiguriert ist zum Erlauben einer Datenübertragung von der Steuereinheit zu der Hochspannungspulsverbindung.

9. Vorrichtung nach einem der vorhergehenden Ansprüche, wobei das Magnetron außerdem mindestens eine umfasst von: einem Permanentmagnet, einem Elektromagnet, Wasserverbindungen zum Kühlen, und einem mechanischen Träger.

10. Vorrichtung nach einem der vorhergehenden Ansprüche, wobei das Magnetron außerdem umfasst:
einen Hochfrequenztuner (HF-Tuner), wobei der HF-Tuner außerhalb des Gehäuses montiert ist und mit der Steuereinheit digital verbunden ist; und
einen Hochfrequenztuner (HF-Tuner), wobei der HF-Tuner außerhalb des Gehäuses montiert ist und über eine Tuner-Antriebsschaltung mit der Steuereinheit digital verbunden ist, und wobei die Glasfaser über die Tuner-Antriebsschaltung mit der Steuereinheit verbunden ist; und
einen Ausgabe-Hochfrequenz-Übergangswellenleiter (Ausgabe-HF-Ausgangsübergangswellenleiter), um das HF-Feld an den Wellenleiter auszugeben, und eine HF-Übergangswellenleiterabdichtung, wobei die HF-Übergangswellenleiterabdichtung ein dielektrisches Gas bereitstellt, um ein Fenster des Wellenleiters zu schützen.

11. Verfahren zum Bereitstellen einer Rückverfolgbarkeit und einer Zustandsüberwachung eines Magnetrons in einer Hochleistungsvorrichtung, wobei das Verfahren umfasst:
Aufzeichnen von Daten, die das Magnetron betreffen, in einem Identifizierungschip, wobei der Identifizierungschip an dem Magnetron angebracht ist;
Lesen der Daten durch eine elektronische Schaltung;
Kommunizieren der Daten zu einer Steuereinheit in der Hochleistungsvorrichtung; und
basierend auf den Daten, Überwachen des Zustands des Magnetrons.

12. Verfahren nach Anspruch 11, wobei das Verfahren außerdem ein Korrelieren der Daten mit mindestens den in der Hochleistungsvorrichtung gespeicherten Daten oder den an einem Cloud-Basisaufbewahrungsort gespeicherten Daten umfasst, wobei die gespeicherten Daten eine Historie des Magnetrons beinhalten.

13. Verfahren nach Anspruch 11 oder Anspruch 12, wobei die Daten mindestens eines umfasst aus: einer eindeutigen Seriennummer; einem Namen eines Herstellers; einem Herstellungsdatum; einer Artikelnummer; einem Bauteilprüfplan; einer Seriennummer des Herstellers, und/oder wobei die Daten über eine Verschlüsselung mit öffentlichem Schlüssel verschlüsselt werden, und wobei die eindeutige Seriennummer Teil eines privaten Schlüssels ist.

14. Verfahren nach einem der Ansprüche 11 bis 13, wobei das Kommunizieren der Daten mit einer Glasfaser durchgeführt wird.

15. Partikelbeschleuniger, umfassend:
einen Wellenleiter zum Beschleunigen von geladenen Partikeln entlang eines Beschleunigungswegs und die Hochleistungsvorrichtung nach einem der Ansprüche 1 bis 10.

## Revendications

1. Dispositif haute puissance pour fournir un champ électromagnétique radiofréquence à un guide d'ondes, le dispositif comprenant :
un magnétron (124) configuré pour fournir un champ électromagnétique radiofréquence à un guide d'ondes ; et, une unité de commande (230) configurée pour commander le magnétron pour délivrer une énergie radiofréquence au guide d'ondes,
le magnétron comprenant :
une connexion à impulsions haute tension (210) enfermée dans un enceinte (222) ;
une connexion de chauffage (212) configurée pour permettre à une connexion électrique de pénétrer dans l'enceinte ; et
un mécanisme configuré pour transmettre des données entre le magnétron et l'unité de commande,
**caractérisé en ce que** le mécanisme comprend :
une puce d'identification (310) configurée pour comporter les données, les données concernant le magnétron ;
un circuit électronique (312) configuré pour lire les données sur la puce d'identification ; et,
un canal de communication (314) configuré pour éloigner les données de la connexion à impulsions haute tension vers l'unité de commande.

2. Dispositif selon la revendication 1, le dispositif comprenant en outre des données stockées sur le dispositif haute puissance et/ou des données stockées sur un dépôt hébergé dans le nuage, les données stockées comportant un historique du magnétron.

3. Dispositif selon la revendication 1 ou 2, le canal de communication étant une connexion de fibre optique.

4. Dispositif selon l'une quelconque des revendications précédentes, le circuit électronique étant monté aux bornes de la connexion de chauffage, et la connexion de chauffage étant configurée pour fournir de la puissance au circuit électronique.

5. Dispositif selon l'une quelconque des revendications précédentes, la connexion de chauffage fournissant une connexion entre le circuit électronique et la puce d'identification.

6. Dispositif selon l'une quelconque des revendications précédentes, l'enceinte comprenant un blindage à compatibilité électromagnétique (EMC).

7. Dispositif selon l'une des revendications précédentes,
les données étant des données à bande passante étroite ; et/ou
les données comprenant : un numéro de série unique et/ou un nom d'un fabricant et/ou une date de fabrication et/ou un numéro de pièce et/ou un calendrier de révision de pièce et/ou un numéro de série de fabricant ; et/ou les données étant cryptées via un cryptage par clé publique, et le numéro de série unique faisant partie d'une clé privée.

8. Dispositif selon la revendication 3, la connexion de fibre optique étant en outre configurée pour permettre une transmission de données de l'unité de commande à la connexion à impulsions haute tension.

9. Dispositif selon l'une quelconque des revendications précédentes, le magnétron comprenant en outre : un aimant permanent et/ou un électroaimant et/ou des raccordements d'eau pour un refroidissement et/ou un support mécanique.

10. Dispositif selon l'une quelconque des revendications précédentes, le magnétron comprenant en outre un accordeur radiofréquence (RF), l'accordeur RF étant monté en dehors de l'enceinte et étant connecté numériquement à l'unité de commande ; et un accordeur radiofréquence (RF), l'accordeur RF étant fixé en dehors de l'enceinte et étant connecté numériquement à l'unité de commande via un circuit de commande d'accordeur, et la fibre optique étant connectée à l'unité de commande via le circuit de commande d'accordeur ;
un guide d'ondes de transition radiofréquence (RF) de sortie pour délivrer le champ RF au guide d'onde, et un joint de guide d'ondes de transition RF, le joint de guide d'ondes de transition RF fournissant un gaz diélectrique pour protéger une fenêtre du guide d'onde.

11. Procédé de fourniture d'une traçabilité et d'une surveillance de condition d'un magnétron dans un dispositif haute puissance, le procédé comprenant :
l'enregistrement de données liées au magnétron dans une puce d'identification, la puce d'identification étant placée sur le magnétron ;
la lecture des données par un circuit électronique ;
la communication des données à une unité de commande dans le dispositif haute puissance ; et
sur la base des données, la surveillance d'une condition du magnétron.

12. Procédé selon la revendication 11, le procédé comprenant en outre la corrélation des données avec des données stockées sur le dispositif haute puissance et/ou des données stockées sur un dépôt hébergé sur le nuage, les données stockées comportant un historique du magnétron.

13. Procédé selon la revendication 11 ou la revendication 12, les données comprenant : un numéro de série unique et/ou un nom d'un fabricant et/ou une date de fabrication et/ou un numéro de pièce et/ou un calendrier de révision de pièce et/ou un numéro de série de fabricant, et/ou les données étant cryptées via un cryptage par clé publique, et le numéro de série unique faisant partie d'une clé privée.

14. Procédé selon l'une quelconque des revendications 11 à 13, la communication des données étant réalisée avec une fibre optique.

15. Accélérateur de particules comprenant :
un guide d'ondes pour accélérer des particules chargées le long d'un chemin d'accélération ; et
le dispositif haute puissance selon l'une quelconque des revendications 1 à 10.
